# EUROPEAN PATENT SPECIFICATION

(11) **EP 1 931 610 B1**
(45) Date of publication and mention of the grant of the patent: **11.11.2009**
(21) Application number: 06802248.2
(22) Date of filing: 24.08.2006
(51) Int. Cl.: C07C 2/28, C07C 29/04

(54) **SELECTIVE OLEFIN OLIGOMERIZATION**
SELEKTIVE OLEFINOLIGOMERISIERUNG
OLIGOMÉRISATION SÉLECTIVE D OLÉFINE

(30) Priority: 07.10.2005 US 245708
(43) Date of publication of application: 18.06.2008
(73) Proprietor: Lyondell Chemical Technology, L.P., Greenville, DE 19807 (US)
(72) Inventor: CANDELA, Lawrence, M., Havertown, Pennsylvania 19083 (US); ZAK, Thomas, S., West Chester, Pennsylvania 19382 (US)
(74) Representative: De Hoop, Eric
(86) International application number: PCT/US2006/033056
(87) International publication number: WO 2007/044137

(56) References cited:
- WO-A1-01/51435
- US-A- 4 100 220
- US-A- 5 382 705

## Description

### BACKGROUND OF THE INVENTION

### FIELD OF THE INVENTION

The present invention relates to a process for the selective oligomerization of olefins such as isobutylene to form mainly the dimer wherein a selectivity enhancing modifier such as t-butanol (TBA) is employed, and especially to an integrated process wherein the modifier is formed in a reaction separate from the oligomerization in a safe and convenient manner and the modifier forming step is integrated into the overall oligomerization procedure.

### DESCRIPTION OF THE PRIOR ART

The oligomerization of olefins such as propylene using acidic catalysts is by now well known in the art.

As described in U.S, 3,760,026, a number of catalysts are known for this reaction including cold sulfuric acid, phosphoric acid on Kieselguhr, silica/alumina sometimes promoted with Ni, Co, Fe, Pt or Pd; activated natural clays plus activating substances such as ZnO, metallic phosphates such as those of iron (III) and cerium optionally supported on carriers such as activated carbon, bauxite, activated carbon alone and with metal halides such as TiCl₂, heteropolyacids such as silicotungstic acid on silica gel and phosophomolybdic acid, BF₃H₃PO₄ and BF₃HPO₃; dihydroxyfluoroboric acid, HF, and fluorides or oxyfluorides of S, Se, N, P, Mo, Te, W, V, and Si boiling below 300 degrees C; and AlCl₃ with cocatalysts such as dimethly ether, HCl and nitromethane. An especially preferred catalyst is a sulfonic acid-type ion exchange resin such as Amberlyst A-15, A-35, A-36, Purolite CT275, and the like. U.S. Patent 4,100,220 is illustrative and teaches use of TBA, water, or mixtures as reaction modifiers. U.S. Patent 4,447,668 describes isobutylene dimerization using A-15 with methyl t-butyl ether as solvent.

U.S. Patent 5,877,372 describes isobutylene dimerization using sulfonic acid resin catalyst, TBA modifier and a diluent such as isooctane. The use of a selectivity enhancing modifier such TBA is important in order to achieve high dimerization selectivity. The addition of water to the dimerization reactor has been suggested, as in U.S. Patent 4,100,220 where the water reacts in the dimerization reactor with isobutylene to form TBA. See also U.S. Patent 6,613,108 which also suggests water addition to the dimerization reactor.

A problem which results from the addition of water to the dimerization reactor is that water tends not to be evenly distributed throughout the catalyst bed and, in the local absence of water, there is a real tendency for uncontrolled runaway dimerization to occur due to the exothermic nature of the reaction.

### SUMMARY OF THE INVENTION

In accordance with the present invention, a process is provided for the oligomerization of an olefin such as isobutylene in the presence of a selectivity enhancing amount of a modifier such as TBA wherein the modifier is formed as part of the overall process but in a reactor separate from the oligomerization reactor and the introduction of water into the oligomerization reactor is substantially avoided.

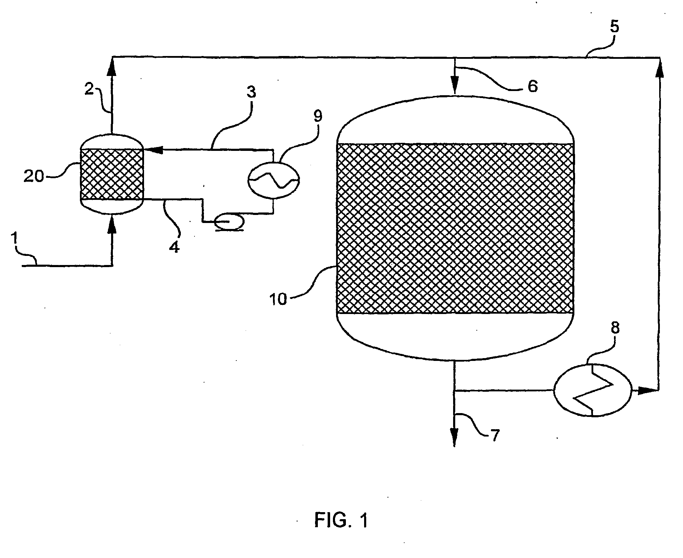

### BRIEF DESCRIPTION OF THE DRAWING

The accompanying drawing is a schematic representation of an especially preferred practice of the invention.

### DETAILED DESCRIPTION

With reference to the drawing and the process represented therein, reactor 10 is a suitable oligomerization reactor which is packed with appropriate catalyst such as a sulfonic acid ion exchange resin. A much smaller reactor 20 is provided which preferably is packed with the same catalyst contained in reactor 10 although it is quite feasible to use less expensive hydration catalyst such as gel-type resins in the hydration reactor.

A feed stream which contains olefin, preferably isobutylene, is fed to reactor 20 via line 1 and water is fed to reactor 20 via line 3. In most effective operation, sufficient water is added to cover the entire bed of solid catalyst thus avoiding the possibility that olefin could contact the catalyst bed in the absence of water which might result in a local runaway reaction. A cooling loop is provided whereby an aqueous stream is withdrawn via line 4, cooled and returned to the reactor in order to remove reaction heat.

Conditions are maintained in reactor 20 effective for the reaction of water with olefin fed via line1 with the formation of an alcohol dimerization selectivity enhancing modifier such as TBA. It will be understood that olefines other then isobutylene can be reacted, including mixtures of olefins such linear C₄ olefins with isobutylene, to form the modifier composition.

The modifier containing product stream which is essentially water-free is passed from reactor 20 to reactor 10 via line 2 where it is admixed with a recycle stream from reactor 10 which has been cooled in heat exchanger 8 to remove heat of reaction. The amount of modifier fed via line 2 is maintained at a level sufficient to provide the desired amount of modifier relative to olefin in oligomerization reactor 10.

It is possible to pass the entire olefin feed to reactor 10 through reactor 20 as indicated in the drawing or the feed olefin can be divided so that only a portion passes through reactor 20 in order to lessen the necessary size of reactor 20.

In reactor 10, conditions are controlled to effect the desired selective oligomerization of the feed olefin. The reaction effluent from reactor 10 passes via line 7 and is divided into a recycle stream which is cooled in heat exchanger 8 before returning to reactor 10 and a product stream which passes via line 7 to dimer recovery (not shown). Conventional recovery means such as distillation are employed to separate product dimer from other materials some of which can be recycled.

### EXAMPLE I

A high purity isobutylene feed such as that resulting from TBA dehydration is employed as the olefin feed in this example. The olefin stream is fed via line 1 to reactor 20 which is packed with A-15 ion exchange resin. Water is added via line 3 to the stream circulating via line 4 to cooler 9 which is used to remove heat of reaction in reactor 20. The temperature in reactor 20 is maintained at about 60 degrees C; generally, temperatures of 40-100 degrees C, preferably 60-8 degrees C are especially useful.

In reactor 20, essentially complete conversion of the net added water to TBA product takes place and the reaction effluent which is the organic phase essentially free of water passes via line 2 and is combined with a recycle stream in line 5 and the combined streams pass via line 6 to dimerization reactor 10. The oligomerization reactor 10 is likewise packed with A-15 sulfonic acid resin catalyst; conditions in reactor 10 include a reaction temperature of about 80-100 degrees C. In reactor 10 the isobutylene is selectively dimerized in the presence of the TBA formed in reactor 20 to form diisobutylene in high selectivity. Effluent from reactor 10 is divided with a portion circulated through cooler 8 back to reactor 10 via lines 5 and 6 and net effluent being recovered via line 7.

The following Table I shows the stream flows at various points in the reaction system, the flows being given in pounds per hour of the various components.

**TABLE I**

| | | Stream flows lbs/hr | | | | | | |
|---|---|---|---|---|---|---|---|---|
| Components | Stream # | 1 | 2 | 3 | 4 | 5 | 6 | 7 |
| Isobutene | | | | | | | | |
| n-butane | | | | | | | | |
| Isobutylene | | 100 | 97 | | | 89.11 | 119.1089 | 30 |
| butene-1 | | | | | | | | |
| cis-butene-2 | | | | | | | | |
| trans-butene-2 | | | | | | | | |
| t-butanol | | | 4 | | 35 | 11.88 | 15.88119 | 4 |
| sec-butanol | | | | | | | | |
| Diisobutylene | | | | | | 188.9 | 252.5109 | 63.6 |
| Dimethyl hexenes | | | | | | 3.267 | 4.367327 | 1.1 |
| Other Trimethyl pentenes | | | | | | 4.455 | 5.9555446 | 1.5 |
| Trimers | | | | | | 2.376 | 3.176238 | 0.8 |
| Tetramer & Hvys | | | | | | | | |
| Other Trimethyl pentenes | | | | | | | | |
| Water | | | | 1 | 200 | | | |
| | | 100 | 101 | | | 300 | 401 | 101 |

### EXAMPLE 2

In this example, in place of the high purity isobutylene feed used in Example 1, the olefin feed is a mixed C₄ refinery stream. Temperatures in reactors 10 and 20 are the same as in Example 1 and the same catalysts are employed. Results obtained are given in the following Table II.

**TABLE II**

| | | Stream flows lbs/hr | | | | | | |
|---|---|---|---|---|---|---|---|---|
| Components | Stream # | 1 | 2 | 3 | 4 | 5 | 6 | 7 |
| isobutene | | 2.4 | 2.4 | | | 7.2 | 9.6 | 2.4 |
| n-butane | | 17.2 | 17.2 | | | 51.6 | 68.8 | 17.2 |
| Isobutylene | | 38.65 | 34.9 | | | 40.05 | 53.4 | 13.35 |
| butene-1 | | 25.4 | 25.4 | | | 72.9 | 97.2 | 24.3 |
| 1,3 butanediol | | 0.9 | 0.9 | | | 1.8 | 2.4 | 0.6 |
| cis-butene-2 | | 6.25 | 6.25 | | | 18 | 24 | 6 |
| trans-butene-2 | | 9.2 | 9.2 | | | 26.1 | 34.8 | 8.7 |
| t-butanol | | 0 | 5 | | 50 | 5.4 | 7.2 | 1.8 |
| sec-butanol | | | | | | 1.71 | 2.28 | 0.57 |
| Diisobutylene | | | | | | 58.5 | 78 | 19.5 |
| Dimethyl hexenes | | | | | | 4.5 | 6 | 1.5 |
| Other Trimethyl pentenes | | | | | | 6 | 8 | 2 |
| sec-Butyl t-butyl ether | | | | | | 5.4 | 7.2 | 1.8 |
| Trimers | | | | | | 4.68 | 6.24 | 1.56 |
| Tetramer & Hvys | | | | | | 0.06 | 0.08 | 0.02 |
| Water | | | | 13 | 185 | 0 | 0 | 0 |
| | | 100 | 101.3 | | | 303.9 | 405.2 | 101.3 |

The reaction conditions employed in reactors 10 and 20 are generally known. In general, known oligomerization and hydration catalysts and conditions can be employed in each step. Suitable conditions include temperatures broadly in the range of 0 to 200 degrees C, preferably 10 to 100 degrees C, and the use of pressures sufficient to maintain the liquid phase, illustratively above 50 psig, e.g. 50-500 psig.

Known catalysts can be used for each step; preferably the same catalyst is used in each of the reaction steps. U.S. Patent 3,760,026 is illustrative. The use of sulfonic acid type ion exchange resins such as Amberlyst A-15, A-35, A-36, Purolite CT275, Dowex 50, and the like is especially preferred.

A feature of the present invention is the use of alcohol such as TBA as selectivity enhancing modifier in the selective olefin oligomerization, which modifier is separately formed, whereby the presence of water in the oligomerization reactor and the accompanying problems associated with uneven distribution of the modifier throughout the reactor are avoided. To this end, the hydration reactor 20 is operated such that the stream passing from reactor 20 to dimerization reactor 10 is essentially water free. It is an important feature that water be essentially excluded from reactor 10 in contrast to procedures favored in the prior art. In the operation of the hydration reactor 20 both aqueous and organic phases are formed and there exists in the reactor an interface between the organic and aqueous phases with the modifier distributed between the phases. Operation is carried out such that the organic phase containing both olefin and modifier is separated via line 2 and sent to the dimerization reactor while the aqueous phase is circulated through cooler 9 to remove reaction exotherm. Flow rates and temperatures in reactor 20 are regulated in accordance with conventional engineering practices to provide sufficient modifier in the stream in line 2 for purposes of dimerization while at the same time the stream is essentially water free, i.e. containing less than about 1 wt.% water.

Various other known dimerization features can be employed such as the use of diluents as described in U.S. Patent 5,877,372.

An outstanding feature of the present invention is the avoidance of the introduction of water into the oligomerization resulting in much safer and uniform selective production of dimer product.

## Claims

1. In a process for the selective oligomerization of an olefin to the dimer in the presence of a selectivity enhancing modifier, the improvement which comprises preparing the modifier in a first reaction by reacting an olefin and water, separating the product modifier substantially free of water and oligomerizing olefin in the presence of said separated modifier to selectively form the dimer.

2. In a process for the selective oligomerization of isobutylene to diisobutylene in the presence of a selectivity enhancing modifier, the improvement which comprises preparing the said modifier in a first reaction by reacting isobutylene and water, separating the product modifier substantially free of water and oligomerizing isobutylene in the presence of said separated modifier to selectively form diisobutylene.

3. The process of Claim 2 wherein said modifier is TBA.

4. In a process for the selective oligomerization of isobutylene to diisobutylene in the presence of a mixture of selectivity enhancing modifiers, the improvement which comprises preparing the modifiers in a first reaction by reacting a hydrocarbon mixture containing mixed C₄ olefins with water to form alcohols, separating the said alcohols substantially free of water, and oligomerizing the mixed C₄ olefins in the presence of said alcohols to selectively form diisobutylene.

5. The process of Claim 1 wherein a sulfonic acid ion exchange resin is used in both reactions.

## Patentansprüche

1. Bei einem Verfahren zur selektiven Oligomerisierung eines Olefins zu dem Dimer in Gegenwart eines die Selektivität verbessernden Modifikators, die Verbesserung, die die Herstellung des Modifikators in einer ersten Reaktion durch Umsetzen von einem Olefin und Wasser, die Abtrennung des im wesentlichen wasserfreien produzierten Modifikators und die Oligomerisierung des Olefins in Gegenwart des abgetrennten Modifikators, um selektiv das Dimer zu bilden, umfasst.

2. Bei einem Verfahren zur selektiven Oligomerisierung von Isobutylen zu Diisobutlyen in Gegenwart eines die Selektivität verbessernden Modifikators, die Verbesserung, die die Herstellung des Modifikators in einer ersten Reaktion durch Umsetzen von Isobutylen und Wasser, die Abtrennung des im wesentlichen wasserfreien produzierten Modifikators und die Oligomerisierung von Isobutylen in Gegenwart des abgetrennten Modifikators, um selektiv Diisobutylen zu bilden, umfasst.

3. Verfahren nach Anspruch 2, wobei der Modifikator TBA ist.

4. Bei einem Verfahren zur selektiven Oligomerisierung von Isobutylen zu Diisobutlyen in Gegenwart eines Gemisch von die Selektivität verbessernden Modifikatoren, die Verbesserung, die die Herstellung des Modifikators in einer ersten Reaktion durch Umsetzen von einem Kohlenwasserstoffgemisch, enthaltend vermischten C₄-Olefine, mit Wasser, um Alkohole zu bilden, die Abtrennung der im wesentlichen wasserfreien Alkohole und die Oligomerisierung der vermischten C₄-Olefine in Gegenwart der Alkohole, um selektiv Diisobutylen zu bilden, umfasst.

5. Verfahren nach Anspruch 1, wobei bei beiden Reaktionen ein Sulfonsäure-Ionenaustauscherharz eingesetzt wird.

## Revendications

1. Dans un procédé pour l'oligomérisation sélective d'une oléfine en le dimère en présence d'un modificateur augmentant la sélectivité, le perfectionnement qui comprend la préparation du modificateur dans une première réaction par réaction d'une oléfine et d'eau, la séparation du modificateur produit sensiblement sans eau et l'oligomérisation de l'oléfine en présence dudit modificateur séparé pour former sélectivement le dimère.

2. Dans un procédé pour l'oligomérisation sélective de l'isobutylène en diisobutylène en présence d'un modificateur augmentant la sélectivité, le perfectionnement qui comprend la préparation du modificateur dans une première réaction par réaction d'isobutylène et d'eau, la séparation du modificateur produit sensiblement sans eau et l'oligomérisation de l'isobutylène en présence dudit modificateur séparé pour former sélectivement du diisobutylène.

3. Procédé selon la revendication 2 où ledit modificateur est le TBA.

4. Dans un procédé pour l'oligomérisation sélective de l'isobutylène en diisobutylène en présence d'un mélange de modificateurs augmentant la sélectivité, le perfectionnement qui comprend la préparation des modificateurs dans une première réaction par réaction d'un mélange d'hydrocarbures contenant des C₄ oléfines mélangées avec de l'eau pour former des alcools, la séparation desdits alcools sensiblement sans eau et l'oligomérisation des C₄ oléfines mélangées en présence desdits alcools pour former sélectivement du diisobutylène.

5. Procédé selon la revendication 1 où une résine échangeuse d'ions de type acide sulfonique est utilisée dans les deux réactions.
